(19) Europäisches Patentamt
European Patent Office
Office européen des brevets

(11) Publication number: **0 202 903**
**A2**

(12) # EUROPEAN PATENT APPLICATION

(21) Application number: 86303783.4

(22) Date of filing: 19.05.86

(51) Int. Cl.[4]: C 07 C 49/80
C 07 C 49/813, C 07 C 79/36
C 07 C 45/46, C 07 C 45/67
A 01 N 35/04, A 01 N 43/10
C 07 D 333/22, C 07 D 333/28
C 07 D 333/42, C 07 D 333/44

(30) Priority: 20.05.85 US 736177

(43) Date of publication of application:
26.11.86 Bulletin 86/48

(84) Designated Contracting States:
AT BE CH DE FR GB IT LI LU NL SE

(71) Applicant: ELI LILLY AND COMPANY
Lilly Corporate Center
Indianapolis Indiana 46285(US)

(72) Inventor: Boisvenue, Rudolf Joseph
329 North State Street
Greenfield Indiana 46140(US)

(72) Inventor: Crouse, Gary Dean
1088 North Shortridge Road
Indianapolis Indiana 46219(US)

(72) Inventor: Kramer, Kenneth Edward
6455 North Oakland Avenue
Indianapolis Indiana 46220(US)

(74) Representative: Tapping, Kenneth George et al,
Erl Wood Manor
Windlesham Surrey, GU20 6PH(GB)

(54) **Control of ectoparasites.**

(57) A series of 1,3-propanediones having a perfluoroalkyl group or a perfluorocycloalkyl group on one carbonyl, and a substituted phenyl or thienyl group on the other, are useful when administered to animals for the control of ectoparasites.

EP 0 202 903 A2

# CONTROL OF ECTOPARASITES

The present invention relates to novel propanedione derivatives, their preparation and use in the control of ectoparasites.

The control of ectoparasites, such as fleas, ticks, biting flies and the like, has long been recognized as an important problem in animal husbandry. The traditional treatments for domestic animals were topically applied insecticides, such as the famous dips for sheep. Indeed, such treatments are still in wide use. However, the more modern thrust of research has been towards compounds which can be administered to the animals, especially orally, and which will control ectoparasites by poisoning the parasite when it ingests the blood of the treated animal.

South African Patent 71/4221 discloses the insecticidal activity of 1,3-propanediones having a trifluoromethyl group on one carbonyl, and a substituted phenyl group on the other carbonyl. Ectoparasiticidal use of some of their compounds is also disclosed.

Canadian Patent No. 862,068 illustrates a group of 1,3-propanediones, having a phenyl or naphthyl group on one carbonyl and a perhalomethyl or perhaloethyl group on the other carbonyl. These compounds are said to be insecticides but there is no reference to the treatment of animals.

U.S. Patent No. 3,636,214 describes 1,3-propanediones having an aryl group, including a thienyl group, on one carbonyl, and a trifluoromethyl or other

haloalkyl group on the other carbonyl. However, this document describes the use of such compounds only for the purpose of controlling fungi, especially in the soil.

The present invention provides a method of controlling a population of insect or acarina ectoparasites which consume blood of a host animal which comprises administering to the host animal an effective amount of a compound of the formula (I):

$$R\text{-}CO\text{-}CH_2\text{-}CO\text{-}R^1 \qquad (I)$$

wherein R is phenyl or thienyl substituted by halo, trifluoromethyl, nitro, chloroethenyl or bromoethenyl;

$R^1$ is $C_3$-$C_6$ perfluoroalkyl or perfluorocycloalkyl;

or a sodium, potassium or lithium salt thereof.

Preferably, the R group has one or two substituents.

The compounds of formula (I), except the free form of those where R is 5-bromo or 5-chloro-thien-2-yl and $R^1$ is $C_3$ perfluoroalkyl, are novel and are provided in one aspect of the invention.

Throughout the present document, all temperatures are expressed in degrees Celsius. All expressions of percentage, concentration and the like are in weight units unless otherwise described.

The term "halo" refers to fluoro, chloro, bromo or iodo. The term "chloroethenyl" or "bromoethenyl" refers to an ethenyl group substituted with one or two chlorine or bromine atoms, respectively, on the terminal carbon atom.

The terms "perfluoroalkyl" and "perfluorocycloalkyl" refer to alkyl or cycloalkyl groups in which all of the hydrogen atoms have been replaced by fluorine atoms. The term is used to avoid the long chemical names that result from naming the locations of the individual fluorine atoms. Thus, the $R_1$ groups of the compounds used in the present invention include perfluoroalkyl and perfluorocycloalkyl groups such as perfluoropropyl, perfluoroisopropyl, perfluoroisopentyl, perfluoro(2-methylpentyl), perfluorocyclopropyl, perfluorocyclohexyl and the like.

R is preferably optionally substituted phenyl, and the preferred $R^1$ group is $C_3$ perfluoroalkyl.

While the compounds used in the present invention are consistently named as 1,3-propanediones in the present document, it will be understood that it is quite possible if not probable that one of the carbonyl groups will actually be in the enol form. That is to say, the compound will exist in a form described as follows.

$$R\text{-}CO\text{-}CH{=}C(OH)\text{-}R^1$$

Indeed, it is most probable that the compounds exist in an equilibrium form containing some of the diketone and some of the enol at all times. A careful study of the equilibrium has not been carried out, and so the compounds will be described here as diketones in all cases. It will be understood by the skilled reader that the principles and function of the present invention remain the same, whether carried out with the diketone, with pure enol, or with an equilibrium mixture of the diketone and the corresponding enol.

It is believed that the nature of the compounds used in the present invention has been made entirely clear. However, to assure that the reader fully comprehends the invention, a group of exemplary compounds will be mentioned here.

1-(4-chloro-3-iodophenyl)-3-perfluoropropyl-1,3-propanedione

1-(3-bromo-5-fluorophenyl)-3-perfluoroisopropyl-1,3-propanedione

1-(2-fluoro-4-trifluoromethylphenyl)-3-perfluorocyclohexyl-1,3-propanedione

1-(3-fluorophenyl)-3-perfluorobutyl-1,3-propanedione

1-(5-nitrothien-2-yl)-3-perfluorocyclopentyl-1,3-propanedione

1-(4-chloro-5-iodothien-3-yl)-3-perfluorocyclopentyl-1,3-propanedione

1-(2-fluoro-5-trifluoromethylthien-3-yl)-3-perfluoro(1-methylpropyl)-1,3-propanedione

1-(3-nitro-5-trifluoromethylphenyl)-3-perfluorocyclopropyl-1,3-propanedione

1-[3-(2,2-dibromoethenyl)phenyl]-3-perfluorocyclobutyl-1,3-propanedione

1-[4-(2,2-dichloroethenyl)phenyl]-3-perfluoropentyl-1,3-propanedione

1-[2-chloro-4-(2,2-dichloroethenyl)phenyl]-3-perfluoro(2-ethylbutyl)-1,3-propanedione

1-[4-bromo-3-(2,2-dibromoethenyl)thien-2-yl]-3-perfluoro(1-methylpentyl)-1,3-propanedione

1-(2,4-diiodophenyl)-3-perfluorocyclopropyl-1,3-propanedione

1-(2,5-difluorophenyl)-3-perfluorocyclopropyl-1,3-propanedione, potassium salt

1-(2,6-dinitrophenyl)-3-perfluoro-*t*-butyl-1,3-propanedione, sodium salt

1-[2,4-bis(trifluoromethyl)phenyl]-3-perfluoropentyl-1,3-propanedione, sodium salt

1-(4,5-difluorothien-2-yl)-3-perfluoro(3-methylbutyl)-1,3-propanedione, lithium salt

1-(3,4-diiodothien-2-yl)-3-perfluorocyclopropyl-1,3-propanedione, potassium salt

1-[3,5-bis(trifluoromethyl)thien-2-yl]-3-perfluorocyclobutyl-1,3-propanedione, lithium salt

1-(2,5-dinitrothien-3-yl)-perfluoropentyl-1,3-propanedione, sodium salt

1-(5-nitro-3-trifluoromethylthien-2-yl)-3-perfluorohexyl-1,3-propanedione, potassium salt

1-(3-chloro-4-nitrothien-2-yl)-3-perfluoropropyl-1,3-propanedione, sodium salt

1-(4-chloro-3-nitrophenyl)-3-perfluoropropyl-1,3-propanedione, potassium salt.

The most preferred compounds for use in the present invention are the following.

1-perfluoropropyl-3-(3-trifluoromethylphenyl)-1,3-propanedione

1-perfluoropropyl-3-(2,4-difluorophenyl)-1,3-propanedione

1-(4-chlorophenyl)-3-perfluoropropyl-1,3-propanedione

1-perfluoropropyl-3-(4-trifluoromethylphenyl)-1,3-propanedione

Use of the above particularly preferred compounds in the form of their salts, especially the sodium salt, is also particularly preferred.

According to one aspect of the invention there is provided a method for preparing a compound of formula (I), which comprises a compound of formula:

$$R\text{-}CO\text{-}CH_3 \qquad (II)$$

with an activated form of the acid of formula:

$$R^1CO_2H \qquad (III)$$

in the presence of a base strong enough to generate the anion of the compound of formula (II).

The activated form of the acid will normally be a $C_{1-4}$ alkyl ester of formula:

$$R^1\text{-}CO_2R^3$$

where $R^3$ is $C_{1-4}$ alkyl, preferably ethyl. However, acid halides or acid anhydrides can also be used.

As stated above the acylation should be carried out in the presence of a base strong enough to generate the anion of the acetyl derivative. Suitable bases which may be mentioned are Group IA hydrides such as sodium hydride, or Group IA alkoxides such as sodium methoxide or ethoxide.

The product of the acylation reaction will be a salt of the compound of formula (I) with the base utilised in the acylation reaction. The propanedione may be produced in free form by reaction of that salt with a strong acid such as hydrochloric acid.

The acylation reaction should be effected at a temperature within the range from 0 to 100°C, preferably from 0°C to room temperature, in an inert organic solvent such as toluene or diethyl ether.

When a perfluorocycloalkyl compound is to be made, a preferred synthesis proceeds through a perfluoroalkyl, preferably trifluoromethyl, compound in the alkali metal salt form. That compound is contacted with a strong base, such as sodium hydride, to form an anion, which is reacted with the acid fluoride of the desired perfluorocycloalkyl carboxylic acid. The reaction is readily carried out at ambient or moderately elevated temperature, as Example 5 illustrates.

Thus, during the above described reaction, the anion:

$$R\text{-}CO\text{-}\overset{(-)}{CH}\text{-}COR^4$$

is generated, wherein $R^4$ represents $C_1$-$C_4$ perfluoroalkyl, preferably trifluoromethyl. This anion then reacts with the acid fluoride of formula:

$$R^1COF$$

where $R^1$ represents a $C_3$-$C_6$ perfluoroalkyl group, to form a compound of formula:

$$\begin{array}{c} R\text{-}CO\text{-}CH\text{-}COR^{1a} \\ | \\ COR^1 \end{array}$$

Hydrolysis of this product with an aqueous mineral acid such as hydrochloric acid yields a compound of formula (I) in which $R^1$ is $C_3$-$C_6$ perfluoroalkyl.

The compounds used in the present invention are highly acidic in nature and readily form alkali metal salts. Therefore, the products of the above-described process are routinely obtained in the salt form. When the free acid form is desired, however, it is necessary only to take the product through an acidic step as the reaction mixture is being worked up. For example, a brief wash with a dilute strong acid, such as dilute hydrochloric acid, readily converts a salt to the free acid.

The following preparative examples are given to assure that the reader can obtain any desired compound for use in the present invention.

## Example 1

1-(5-Chlorothien-2-yl)-3-perfluoropropyl-1,3-propanedione

To a 2-liter flask were added 250 ml. of toluene, 45 g. of sodium methoxide and 120 g. of 2-acetyl-5-chlorothiophene. The mixture was cooled to 13°, and was stirred for 30 minutes. To it was then added a solution of 182 g. of ethyl perfluorobutyrate in 250 ml. of dry toluene. The temperature increased to 42°, and the mixture was stirred without heating or cooling for 16 hours. The mixture was then poured slowly into 600 ml. of trifluoroacetic acid. That mixture was then poured into 5 l. of ice-water, and the organic layer was separated. The aqueous layer was extracted twice with 1-liter portions of dichloromethane, and all of the organic layers were combined, washed with brine, dried over sodium sulfate, filtered, and evaporated to dryness under vacuum. The residue, amounting to 201 g., was purified over a silica gel column using 2.5 kg. of silica gel and 8 l. of 1:1 toluene:hexane. The product-containing portions were combined and evaporated to dryness to obtain 106 g. of the desired product, m.p. 41-42°.

## Example 2

1-(5-Bromothien-2-yl)-3-perfluoropropyl-1,3-propanedione

In a 250 ml. flask was mixed 2.9 g. of sodium methoxide and 20 ml. of toluene. To this was added 10 g. of 2-acetyl-5-bromothiophene, washed in with 10 ml. of toluene. Then 13 g. of ethyl perfluorobutyrate dissolved in 20 ml. of toluene was added over a period of 15 minutes, and the mixture was stirred for 36 hours at ambient temperature. It was then poured into 50 ml. of trifluoroacetic acid with stirring, and the mixture was stirred for 30 minutes and then allowed to stand for 1.5 hours. The flask was then filled with water, and the mixture stood for 2 hours more. The organic layer was then separated, and the aqueous layer was washed with two 100 ml. portions of dichloromethane. All of the organic layers were combined and purified as described in Example 1 to obtain 8.4 g. of the desired product.

## Example 3

1-(2,5-Dichlorothien-3-yl)-3-perfluoropropyl-1,3-propanedione

The process of Example 2 was followed, starting with 3 g. of sodium methoxide, 10 g. of 3-acetyl-2,5-dichlorothiophene and 13.7 g. of ethyl perfluorobutyrate, to obtain 2.9 g. of the desired product.

## Example 4

1-(4-Nitrophenyl)-3-perfluoropropyl-1,3-propanedione, sodium salt

Sodium ethoxide was prepared by the reaction of 2.9 g. of 50% sodium hydride and 3.5 ml. of ethanol in 100 ml. of diethyl ether, and to it, at 0°, was added 9.5 g. of ethyl perfluorobutyrate. Then 5 g. of 4-nitroacetophenone was added, and the mixture was stirred for 6 hours. The mixture was then poured onto ice with 50 ml. of 10% disodium hydrogen phosphate solution, and the organic layer was separated, washed with brine, dried, and concentrated under vacuum. The residue was recrystallized from ether:hexane to obtain 9 g. of the desired product, m.p. 190-203°. Its nuclear magnetic spectrum on a 250 mHz instrument showed a singlet at δ6.35, and doublets at δ8.1 and 8.3, J = 8Hz in both cases.

## Example 5

1-(5-Chlorothien-2-yl)-3-perfluorocyclohexyl-1,3-propanedione

A 27.8 g. portion of 1-(5-chlorothien-2-yl)-3-trifluoromethyl-1,3-propanedione, sodium salt, was dissolved in 100 ml. of tetrahydrofuran at ambient temperature. Eight g. of sodium hydride 60% dispersion was washed free of mineral oil, and the hydride was added to the solution, followed by 35 g. of perfluoro-

cyclohexylcarbonyl fluoride, dropwise. The mixture was stirred for one hour, and was poured over ice and the pH of the mixture was adjusted to 1 with 2N hydrochloric acid. The mixture was stirred for 2 hours, and was then extracted with 200 ml. of diethyl ether. The organic layer was dried and concentrated under vacuum, and the residue was chromatographed over silica gel, eluting with hexane. The product-containing fractions were combined and concentrated, and the residue was recrystallized from hexane with cooling to obtain 21 g. of the desired product, m.p. 65°. Its 250 mHz nuclear magnetic resonance spectrum showed a broad singlet at δ11.0, and doublets at δ6.5, J = 4Hz, 7.0, J = 3Hz; and 7.7, J = 3Hz.

Example 6

1-(5-Iodothien-2-yl)-3-perfluoropropyl-1,3-propanedione

Sodium methoxide was prepared from 1.05 g. of sodium hydride, 50% dispersion, in 50 ml. of diethyl ether. To it was added 4.8 g. of ethyl perfluorobutyrate in 25 ml. of ether, and then 5.0 g. of 2-acetyl-5-iodothiophene was washed in with 20 ml. of additional ether. The mixture was stirred at ambient temperature for 2 hours, and was then poured into 50 ml. of trifluoroacetic acid, and the work-up was completed as described in Example 2 above, to obtain 7.4 g. of the desired product, m.p. 32-33°.

## Example 7

1-(4,5-Dichlorothien-2-yl)-3-perfluoropropyl-1,3-propanedione

One g. of 50% sodium hydride dispersion was converted to sodium ethoxide in diethyl ether, and to it was added 2.7 g. of ethyl perfluorobutyrate dissolved in 20 ml. of diethyl ether. The mixture was cooled to 0°, and to it was added 2.0 g. of 2-acetyl-4,5-dichlorothiophene in 20 ml. of ether, dropwise. The mixture was stirred at 0° for 1 hour, and was then poured into a large amount of 10% ammonium chloride solution and ice. The aqueous mixture was extracted with 100 ml. of ethyl acetate, and the organic layer was washed with brine, dried over magnesium sulfate, filtered, and evaporated to dryness to obtain 4.5 g. of crude product. It was dissolved in the minimum amount of chloroform, and was then diluted 5x with dichloromethane. The slurry was filtered to obtain 2.2 g. of the sodium salt of the desired product, m.p. 135-137°. It was dissolved in 1N hydrochloric acid and ethyl acetate, and the organic layer was separated, dried over magnesium sulfate, filtered and evaporated to dryness to obtain 1.8 g. of the desired product, m.p. 27°. Its mass spectrograph showed a molecular ion of weight 390.

## Example 8

1-(4-Chlorophenyl)-3-perfluoropropyl-1,3-propanedione

The process of Example 7 was used, starting with 12.4 g. of sodium hydride dispersion, 46.9 g. of ethyl perfluorobutyrate and 20 g. of 4-chloroacetophenone, to obtain 36.8 g. of the desired product, m.p. 27°. Its 250 mHz NMR spectrum showed a singlet at δ6.6; a broad singlet at δ15.2; and doublets at δ7.5, J = 9Hz; and 7.9, J = 9Hz.

## Example 9

1-[5-(2,2-Dichloroethenyl)thien-2-yl]-3-perfluoropropyl-1,3-propanedione, sodium salt

An 0.5 g. portion of 50% sodium hydride dispersion was converted to sodium ethoxide in 20 ml. of diethyl ether, and to it, at about 0°, was added 1.82 g. of ethyl perfluorobutyrate in 20 ml. of diethyl ether, followed by the dropwise addition of 1.1 g. of 2-acetyl-5-(2,2-dichloroethenyl)thiophene in 50 ml. of ether. The temperature was held at 0-5° with stirring for about 1 hour, and the reaction mixture was then poured into 10% ammonium chloride solution and ice. The aqueous mixture was extracted with 100 ml. of ethyl acetate, and the organic layer was dried over magnesium sulfate, filtered, and evaporated under vacuum to obtain

2.4 g. of crude product, which was purified by chromatography on 30 g. of silica gel, eluting with ethyl acetate. The product-containing fractions were combined and evaporated to obtain 1.7 g. of the desired product, m.p. 170-175°. Its 250 mHz NMR spectrum showed singlets at δ6.22 and 7.6, and doublets at δ7.35, J = 3Hz; and 7.78, J = 3Hz.

## Example 10

1-(5-Chlorothien-2-yl)-3-perfluoropropyl-1,3-propanedione, sodium salt

Sodium ethoxide was prepared from 3.5 g. of sodium hydride at 0° in diethyl ether, and to it was added 10 g. of ethyl perfluorobutyrate followed by 5.3 g. of 2-acetyl-5-chlorothiophene. When the additions were complete, the mixture was allowed to warm to ambient temperature while being stirred for 3 hours, and was then poured over ice and 100 ml. of 10% disodium hydrogen phosphate solution. The organic layer was separated, dried and evaporated under vacuum, and the residue was recrystallized from diethyl ether:hexane to obtain 10.5 g. of the desired product, m.p. 270°. Nuclear magnetic resonance analysis of the product in DMSO showed broad singlets at δ7.05, 7.2, 7.5 and 7.8 and singlets at δ5.6 and 6.2.

## Example 11

1-(4-Chlorophenyl)-3-perfluoropropyl-1,3-propanedione, sodium salt

The process of Example 10 was followed, starting with 9.1 g. of 50% sodium hydride dispersion, 11.6 g. of 4-chloroacetophenone and 24 g. of ethyl perfluorobutyrate, to obtain 22 g. of the desired product, m.p. 220-225°. Its nuclear magnetic resonance spectrum, taken on a 250mHz instrument, showed a singlet at δ6.25, a doublet at δ7.5, J = 8Hz, and a doublet at δ7.9, J = 8Hz.

## Example 12

1-Perfluoropropyl-3-(3-trifluoromethylphenyl)-1,3-propanedione, sodium salt

Sodium methoxide was prepared from 3.5 g. of sodium hydride dispersion in diethyl ether at 0°, and to it was added 10 g. of ethyl perfluorobutyrate and 6.2 g. of 3-trifluoromethylacetophenone. The mixture was stirred for 3 hours at 0°, and was then poured into 50 ml. of 10% ammonium chloride solution and ice. The organic layer was washed with brine, dried and evaporated under vacuum to obtain white impure product, which was recrystallized from diethyl ether:hexane to obtain 11.5 g. of the desired product, m.p. 140-160°. Its nuclear magnetic resonance spectrum, taken at 250 mHz showed a singlet at δ6.3 and a multiplet at δ7.6-8.2.

## Example 13

1-Perfluoropropyl-3-(4-trifluoromethylphenyl)-1,3-propanedione, sodium salt

## Example 14

1-Perfluoropropyl-3-(4-trifluoromethylphenyl)-1,3-propanedione

The process of Example 12 was followed, starting with 3.5 g. of 50% sodium hydride, 6.2 g. of 4-trifluoromethylacetophenone and 10 g. of ethyl perfluorobutyrate to obtain 4.7 g. of the product of Example 13, m.p. 160-180°. Its 250 mHz nuclear magnetic resonance spectrum showed a singlet at δ6.3, a doublet at δ7.8, J = 7Hz and a doublet at δ8.0, J =7Hz.

The filtrate from the process of Example 13 was stirred with 1N hydrochloric acid, and the mixture was extracted with diethyl ether, dried and concentrated under vacuum. The residue was recrystallized from hexane to obtain 0.9 g. of the product of Example 14, m.p. 25°. Its 250 mHz nuclear magnetic resonance spectrum showed a singlet at δ6.6, a doublet at δ7.8, J = 7Hz, a doublet at δ8.0, J = 7Hz, and a broad singlet at δ14.8.

Example 15

1-(2,4-Difluorophenyl)-3-perfluoropropyl-1,3-propanedione, sodium salt

The above product m.p. 145-155°C was prepared by processes similar to those described above.

Example 16

1-Perfluoropropyl-3-(3,5-bis(trifluoromethyl)phenyl)-1,3-propanedione, sodium salt.

2 g. of 50% sodium hydride dispersion was converted with ethanol to sodium ethoxide in diethyl ether, and to it, dropwise, was added 4.8.g. of ethyl perfluorobutyrate and then 5 g. of 3,5-bis(trifluoromethyl)acetophenone at 0°. The mixture was stirred for three hours at 0°, and was worked up as in Example 12 to obtain 2.5 g. of the desired product, M.P. 162-175°.

Representative compounds have been tested in laboratory and target animals to determine the scope of their activity. The following tests are illustrative.

## Test 1

### Guinea pig systemic test

Adult guinea pigs were used in this test system. The compounds to be tested were dissolved in aqueous polyvinyl pyrrolidone or in polyethylene glycol 200, and an appropriate amount of the solution was administered by intraperitoneal injection. Various doses of the compound were used, as named in the tables below.

Blood was drawn from the guinea pigs at different times, shown in the tables below, and the samples of blood were centrifuged. Dental wicks were saturated with the blood serum, and were then exposed in Petri dishes to adult stable flies or adult house flies; blow fly larvae were exposed in test tubes. Various species were used in various tests. After 24 hours, the insects were examined, and the number of dead were counted. The results of the tests were recorded as active, having killed 50% or more of the insects, or inactive, killing less than 50% of the insects.

The following table reports tests of representative compounds. The compounds are identified by their example numbers above. The results are shown by A, indicating activity, and I, indicating inactivity. Some compounds have been tested numerous times under the same conditions. In most such cases, only representative results are reported.

The word "dead" in the table indicates that the guinea pig died before its blood was sampled.

TABLE I

| Compound of Example No. | Dose mg./kg. | Hours | Blow fly | House fly | Stable fly |
|---|---|---|---|---|---|
| 1 | 100 | 24 | Dead | | |
| 1 | 50 | 2 | A | | |
| 1 | 50 | 4 | A | | |
| 1 | 50 | 4 | Dead | | |
| 1 | 50 | 5 | A | I | I |
| 1 | 50 | 6 | A | | |
| 1 | 50 | 6 | Dead | | |
| 1 | 50 | 24 | A | | |
| 1 | 50 | 24 | A | A | A |
| 1 | 50 | 24 | Dead | | |
| 1 | 40 | 6 | Dead | | |
| 1 | 30 | 6 | Dead | | |
| 1 | 25 | 2 | A | | |
| 1 | 25 | 2 | A | A | I |
| 1 | 25 | 4 | A | | |
| 1 | 25 | 4 | Dead | | |
| 1 | 25 | 5 | A | | |
| 1 | 25 | 6 | A | A | A |
| 1 | 25 | 6 | A | | |
| 1 | 25 | 6 | Dead | | |
| 1 | 25 | 24 | A | | |
| 1 | 25 | 24 | A | A | I |
| 1 | 25 | 30 | A | A | A |
| 1 | 25 | 48 | A | A | I |
| 1 | 10 | 2 | A | I | I |
| 1 | 10 | 2 | I | | |
| 1 | 10 | 4 | I | I | I |
| 1 | 10 | 4 | A | | |
| 1 | 10 | 5 | A | I | I |
| 1 | 10 | 5 | A | A | I |
| 1 | 10 | 6 | A | A | |
| 1 | 10 | 6 | A | I | |
| 1 | 10 | 6 | I | | |
| 1 | 10 | 6 | A | | |
| 1 | 10 | 6 | Dead | | |
| 1 | 10 | 6 | A | A | I |
| 1 | 10 | 24 | A | | |
| 1 | 10 | 24 | A | I | |
| 1 | 10 | 24 | A | A | |

TABLE I cont'd.

| Compound of Example No. | Dose mg./kg. | Hours | Blow fly | House fly | Stable fly |
|---|---|---|---|---|---|
| 1 | 10 | 24 | Dead | | |
| 1 | 10 | 30 | I | I | I |
| 1 | 10 | 48 | A | | |
| 1 | 10 | 48 | Dead | | |
| 1 | 10 | 56 | A | | |
| 1 | 7.5 | 6 | I | | |
| 1 | 7.5 | 24 | A | | |
| 1 | 5 | 2 | I | | |
| 1 | 5 | 4 | I | | |
| 1 | 5 | 6 | I | | |
| 1 | 5 | 6 | A | | |
| 1 | 5 | 24 | A | | |
| 1 | 5 | 24 | I | | |
| 1 | 5 | 24 | Dead | | |
| 1 | 2.5 | 6 | I | | |
| 1 | 2.5 | 24 | I | | |
| 2 | 50 | 6 | I | | |
| 2 | 50 | 6 | A | | |
| 2 | 50 | 24 | A | | |
| 2 | 50 | 24 | I | | |
| 2 | 25 | 6 | A | | |
| 2 | 25 | 24 | A | | |
| 2 | 25 | 24 | I | | |
| 2 | 10 | 6 | I | | |
| 2 | 10 | 24 | I | | |
| 3 | 100 | 6 | I | | |
| 3 | 100 | 24 | I | | |
| 3 | 50 | 6 | I | | |
| 3 | 50 | 24 | I | | |
| 3 | 25 | 6 | I | | |
| 3 | 25 | 6 | A | | |
| 3 | 25 | 6 | Dead | | |
| 3 | 25 | 24 | A | | |
| 3 | 25 | 24 | Dead | | |
| 3 | 25 | 24 | I | | |
| 3 | 12.5 | 6 | A | | |
| 3 | 12.5 | 24 | I | | |
| 3 | 10 | 24 | Dead | | |
| 3 | 10 | 24 | I | | |

TABLE I cont'd.

| Compound of Example No. | Dose mg./kg. | Hours | Blow fly | House fly | Stable fly |
|---|---|---|---|---|---|
| 4 | 100 | 2 | A | I | I |
| 4 | 100 | 5 | A | I | I |
| 4 | 100 | 24 | Dead | | |
| 4 | 50 | 2 | A | I | I |
| 4 | 50 | 5 | A | I | I |
| 4 | 50 | 24 | I | I | I |
| 4 | 50 | 48 | I | I | I |
| 5 | 25 | 2 | A | I | I |
| 5 | 25 | 5 | Dead | | |
| 5 | 10 | 2 | I | I | I |
| 5 | 10 | 5 | I | I | I |
| 5 | 10 | 24 | I | A | I |
| 5 | 10 | 48 | I | I | I |
| 6 | 25 | 24 | A | I | |
| 6 | 10 | 6 | I | I | |
| 6 | 10 | 24 | I | I | |
| 7 | 25 | 2 | I | I | I |
| 7 | 25 | 6 | I | I | I |
| 7 | 25 | 24 | A | I | I |
| 7 | 25 | 48 | A | I | I |
| 7 | 10 | 2 | A | I | I |
| 7 | 10 | 6 | A | I | I |
| 7 | 10 | 24 | Dead | | |
| 8 | 100 | 2 | Dead | | |
| 8 | 50 | 2 | Dead | | |
| 8 | 50 | 24 | A | A | A |
| 8 | 50 | 48 | A | I | A |
| 8 | 25 | 2 | A | A | A |
| 8 | 25 | 2 | Dead | | |
| 8 | 25 | 5 | A | A | A |
| 8 | 25 | 24 | A | A | A |
| 8 | 25 | 48 | Dead | | |
| 8 | 10 | 2 | A | A | A |
| 8 | 10 | 2 | A | A | I |
| 8 | 10 | 5 | Dead | | |
| 8 | 10 | 24 | A | I | A |
| 8 | 10 | 24 | I | I | I |
| 8 | 10 | 48 | I | I | I |
| 8 | 5 | 2 | I | I | I |
| 8 | 5 | 6 | I | I | I |
| 8 | 5 | 24 | I | I | I |
| 8 | 5 | 48 | I | I | I |

TABLE I cont'd.

| Compound of Example No. | Dose mg./kg. | Hours | Blow fly | House fly | Stable fly |
|---|---|---|---|---|---|
| 9 | 100 | 2 | A | I | I |
| 9 | 100 | 6 | A | I | A |
| 9 | 100 | 24 | I | I | I |
| 9 | 100 | 48 | I | I | I |
| 9 | 50 | 2 | A | I | A |
| 9 | 50 | 6 | A | I | I |
| 9 | 50 | 24 | I | I | I |
| 9 | 50 | 48 | I | I | I |
| 10 | 10 | 2 | A | A | I |
| 10 | 10 | 2 | I | I | I |
| 10 | 10 | 5 | A | A | I |
| 10 | 10 | 5 | A | I | I |
| 10 | 10 | 24 | I | A | I |
| 10 | 10 | 24 | A | I | I |
| 10 | 10 | 48 | I | I | I |
| 10 | 10 | 48 | A | I | I |
| 10 | 5 | 2 | I | I | I |
| 10 | 5 | 5 | I | I | I |
| 10 | 5 | 24 | I | I | I |
| 10 | 5 | 48 | I | I | I |
| 12 | 25 | 2 | A | A | I |
| 12 | 25 | 6 | Dead | | |
| 12 | 25 | 24 | A | A | A |
| 12 | 25 | 48 | Dead | | |
| 12 | 10 | 2 | A | A | I |
| 12 | 10 | 5 | A | A | I |
| 12 | 10 | 24 | I | I | I |
| 12 | 10 | 48 | I | I | I |
| 12 | 5 | 2 | I | I | I |
| 12 | 5 | 5 | I | I | I |
| 12 | 5 | 24 | I | I | I |
| 12 | 5 | 48 | I | I | I |
| 13 | 25 | 2 | Dead | | |
| 13 | 10 | 2 | A | A | I |
| 13 | 10 | 6 | A | A | I |
| 13 | 10 | 24 | A | I | I |
| 13 | 10 | 48 | I | I | I |
| 14 | 25 | 2 | A | | I |
| 14 | 25 | 6 | Dead | | |

TABLE I cont'd.

| Compound of Example No. | Dose mg./kg. | Hours | Blow fly | House fly | Stable fly |
|---|---|---|---|---|---|
| 14 | 10 | 2 | A | | I |
| 14 | 10 | 6 | Dead | | |
| 14 | 5 | 2 | I | I | I |
| 14 | 5 | 5 | Dead | | |
| 14 | 5 | 24 | I | I | I |
| 14 | 5 | 48 | Dead | | |

The method of the present invention is carried out by administering a compound described above to host animals to control insect and acarina parasites. Administration to the animal may be by the dermal, oral or parenteral routes.

Parasitic insects and acarina include species that are bloodsucking as well as flesh eating and are parasitic during all of their life cycle or only part of their life cycle, such as only the larval or only the adult stage. Representative species include the following:

| | |
|---|---|
| horse fly | _Tabanus_ spp. |
| stable fly | _Stomoxys calcitrans_ |
| black fly | _Simulium_ spp. |
| horse sucking louse | _Haematopinus asini_ |
| mange mite | _Sarcoptes scabiei_ |
| scab mite | _Psoroptes equi_ |
| horn fly | _Haematobia irritans_ |
| cattle biting louse | _Bovicola bovis_ |
| shortnosed cattle louse | _Haematopinus eurysternus_ |
| longnosed cattle louse | _Linognathus vituli_ |
| tsetse fly | _Glossina_ spp. |
| cattle follicle mite | _Demodex bovis_ |
| cattle tick | _Boophilus microplus_ and _B. decoloratus_ |
| Gulf Coast tick | _Amblyomma maculatum_ |
| Lone Star tick | _Amblyomma americanum_ |
| ear tick | _Otobius megnini_ |
| Rocky Mountain wood tick | _Dermacentor andersoni_ |

| | |
|---|---|
| screwworm fly | Cochliomyia hominivorax |
| assassin bug | Reduvius spp. |
| mosquito | Culiseta inornata |
| brown ear tick | Rhipicephalus appendiculatus |
| African red tick | Rhipicephalus evertsi |
| bont tick | Amblyomma sp. |
| bont legged tick | Hyalomma sp. |
| hog louse | Haematopinus suis |
| chigoe | Tunga penetrans |
| body louse | Haematopinus ovillus |
| foot louse | Linognathus pedalis |
| sheep ked | Melophagus ovinus |
| sheep scab mite | Psoroptes ovis |
| greenbottle fly | Phaenicia sericata |
| black blow fly | Phormia regina |
| secondary screw-worm | Cochliomyia macellaria |
| sheep blow fly | Phaenicia cuprina |
| bed bug | Cimex lectularius |
| Southern chicken flea | Echidnophaga gallinacea |
| fowl tick | Argas persicus |
| chicken mite | Dermanyssus gallinae |
| scalyleg mite | Knemidokoptes mutans |
| depluming mite | Knemidokoptes gallinae |
| dog follicle mite | Demodex canis |
| dog flea | Ctenocephalis canis |
| American dog tick | Dermacentor variabilis |
| brown dog tick | Rhipicephalus sanguineus |

The method of the invention may be used to protect economic and companion animals from ectoparasites. For example, the compounds may beneficially be administered to horses, cattle, sheep, pigs, goats, dogs, cats and the like, as well as to exotic animals such as camels, llamas, deer and other species which are commonly referred to as wild animals. The compounds may also beneficially be administered to poultry and other birds, such as turkeys, chickens, ducks and the like. Preferably, the method is applied to economic animals, and most preferably to cattle and sheep.

The rate, timing, and manner of effective application will vary widely with the identity of the parasite, the degree of parasiticidal attack, and other factors. Applications can be made periodically over the entire lifespan of the host, or for only a peak season of parasitic attack. In general, ectoparasite control is obtained with topical application of liquid formulations containing from about 0.00005 to 5.0% of compound, and preferably from about 0.00005 to about 1.0% of compound. Effective parasite control is achieved at administration rates of from about 5 to about 100 mg./kg.

The compounds are applied to host animals by conventional veterinary practices. Usually, the compounds are formulated into ectoparasiticidal compositions which comprise a compound and a physiologically-acceptable carrier. For example, liquid compositions may be simply sprayed on the animals for which ectoparasiticidal control is desired. The animals may also treat themselves by such devices as back rubbers, which

may contain the toxicant compound in a cloth, for example, which the animal may walk against and contact. Dip tanks are also employed to administer the active agent to the host animal.

The present compounds display systemic ectoparasiticidal activity. The compounds have the ability to permeate the tissues of a host animal to which one of the compounds has been administered. Insect parasites which then consume blood or other living tissues of the host animal are thereby killed. The compounds are administered by dermal, oral or percutaneous routes and are preferably formulated prior to administration. Such formulations are well known to those skilled in the art, for example by dissolving the compound in one of many physiologically-acceptable adjuvants or diluents. Oral administration may be performed by mixing the compound in the animals' feed or drinking water, or by administering dosage forms such as tablets, capsules, boluses, or implants. Percutaneous administration is conveniently accomplished by subcutaneous, intraperitoneal and intravenous injection of an injectable formulation.

The compounds can be formulated for oral administration in the usual forms, such as drenches, tablets, or capsules. Such compositions, of course, require orally-acceptable inert carriers. The compounds can also be formulated as an injectable solution or suspension, for subcutaneous, dermal, intraperitoneal, intramuscular, or intravenous injection. In some applications, the compounds are conveniently

formulated as one component of a standard animal feed. In this embodiment, it is usual to formulate the present compound first as a premix in which the compound is dispersed in a liquid or particulate solid carrier. The premix can contain from about 2 to 250 grams of compound per pound. The premix is in turn formulated into the ultimate feed by conventional mixing.

Since ectoparasitic attack generally takes place during a substantial portion of the host animal's lifespan, it is preferred to administer the compounds of the present invention in a form to provide sustained release over a period of time. Conventional procedures include the use of a matrix which physically inhibits dissolution, where the matrix is a waxy semisolid such as the vegetable waxes or a high molecular weight polyethylene glycol. A good way to administer the compounds is by means of a sustained-action bolus, such as those of Laby, U.S. Patent 4,251,506, and Eli Lilly & Co., British Patent 2,059,767. For such a bolus, the compound would be encapsulated in a polymeric matrix such as that of Nevin, U.S. Patent 4,273,920. Sustained release of the compounds of the present invention can also be achieved by the use of an implant such as from a silicone-containing rubber.

The following exemplary compositions illustrate the sort of formulations used to practice the method of the present invention.

Feed Premix

| | |
|---|---|
| Compound of Example 1 | 10% |
| Rice hulls | 85 |
| Light mineral oil | 5 |

Feed Premix

| | |
|---|---|
| Compound of Example 4 | 25% |
| Alfalfa meal | 60 |
| Powdered clay | 5 |
| Molasses | 10 |

Suspension

| | |
|---|---|
| Compound of Example 2 | 30% |
| Naphthalenesulfonate salt | 5 |
| Nonionic surfactant | 5 |
| Fumed silica | 1 |
| Water | 59 |

Drip-On Solution

| | |
|---|---|
| Compound of Example 9 | 20% |
| Nonionic surfactant | 0.8 |
| Propylene glycol | 15 |
| Water | 64.2 |

Drip-On Suspension

| | |
|---|---|
| Compound of Example 6 | 10 |
| Nonionic surfactant | 1 |
| Light mineral oil | 89 |

Injectable Solution

| | |
|---|---|
| Compound of Example 14 | 15% |
| Propylene glycol | 85 |

Injectable Suspension

| | |
|---|---|
| Compound of Example 13 | 25% |
| Propylene glycol | 15 |
| Water | 60 |

Injectable Suspension

| | |
|---|---|
| Compound of Example 8 | 30% |
| Polyvinylpyrrolidone | 2 |
| Water | 68 |

CLAIMS

1. A method of controlling a population of insect or acarina ectoparasites which consume blood of a host animal which comprises administering to the host animal an effective amount of a compound of the formula

$$R\text{-}CO\text{-}CH_2\text{-}CO\text{-}R^1 \quad (I)$$

wherein R is phenyl or thienyl substituted by halo, trifluoromethyl, nitro, chloroethenyl or bromoethenyl;

$R^1$ is $C_3$-$C_6$ perfluoroalkyl or perfluorocycloalkyl;

or a sodium, potassium or lithium salt thereof.

2. A method according to claim 1, wherein the R group has one or two substituents.

3. A method according to claim 1 or 2, wherein the compound of formula (I) is a compound wherein $R^1$ is $C_3$ perfluoroalkyl.

4. A method according to any one of claims 1 to 3, wherein the compound of formula (I) is a compound, wherein R is substituted phenyl.

5. An ectoparasiticidal composition comprising a physiologically-acceptable inert carrier and a compound of formula (I) as defined in any one of claims 1 to 4.

6. An ectoparasiticidal composition as claimed in claim 5 in the form of a feed premix wherein the inert carrier comprises an orally-acceptable inert carrier.

7. A compound of formula (I) as defined in any one of claims 1 to 4, provided that when R is 5-bromo or 5-chlorothien-2-yl and $R^1$ is $C_3$ perfluoroalkyl, then the compound of formula (I) must be in the form of the sodium, lithium or potassium salt.

8. A compound of formula (I) as claimed in claim 7, wherein the R group has one or two substituents.

9. A compound of formula (I) as claimed in claim 7 or 8, wherein R is substituted phenyl and $R^1$ is $C_3$ perfluoroalkyl.

10. 1-Perfluoropropyl-3-(3-trifluoromethylphenyl)-1,3-propanedione, or a sodium salt thereof.

11. 1-Perfluoropropyl-3-(4-trifluoromethylphenyl)-1,3-propanedione, or a sodium salt thereof.

12. 1-Perfluoropropyl-3-(2,4-difluorophenyl)-1,3-propanedione, or a sodium salt thereof.

13. 1-Perfluoropropyl-3-(3,5-bis(trifluoromethyl)-phenyl)-1,3-propanedione, or a sodium salt thereof.

14. A process for preparing a compound of formula (1) as claimed in any one of claims 7 to 13, which comprises:

(A) acylating a compound of formula (II):

$$R\text{-}CO\text{-}CH_3 \qquad (II)$$

with an activated form of the acid of formula (III):

$$R^1CO_2H \qquad (III)$$

in the presence of a base sufficiently strong to generate the anion of the compound of formula (II); or

(B) hydrolyzing a compound of formula:

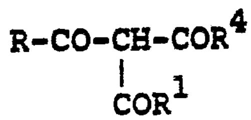

where $R^4$ represents $C_1$-$C_4$ perfluoroalkyl, and $R^1$ represents $C_3$-$C_6$ perfluorocycloalkyl, so as to provide a compound of formula (I) in which $R^1$ is $C_3$-$C_6$ perfluorocycloalkyl.

CLAIMS

1. A method of controlling a population of insect or acarina ectoparasites which consume blood of a host animal which comprises administering to the host animal an effective amount of a compound of the formula

$$R\text{-}CO\text{-}CH_2\text{-}CO\text{-}R^1 \quad (I)$$

wherein R is phenyl or thienyl substituted by halo, trifluoromethyl, nitro, chloroethenyl or bromoethenyl;

$R^1$ is $C_3$-$C_6$ perfluoroalkyl or perfluorocycloalkyl;

or a sodium, potassium or lithium salt thereof.

2. A method according to claim 1, wherein the R group has one or two substituents.

3. A method according to claim 1 or 2, wherein the compound of formula (I) is a compound wherein $R^1$ is $C_3$ perfluoroalkyl.

4. A method according to any one of claims 1 to 3, wherein the compound of formula (I) is a compound, wherein R is substituted phenyl.

5. An ectoparasiticidal composition comprising a physiologically-acceptable inert carrier and a compound of formula (I) as defined in any one of claims 1 to 4.

6. An ectoparasiticidal composition as claimed in claim 5 in the form of a feed premix wherein the inert carrier comprises an orally-acceptable inert carrier.

7. An ectoparasiticidal composition as claimed in claim 5 or 6, wherein the compound of formula (I) is one in which the R group has one or two substituents.

8. An ectoparasiticidal composition as claimed in claim 5, 6 or 7, wherein the compound of formula (I) is one wherein R is substituted phenyl and $R^1$ is $C_3$ perfluoroalkyl.

9. An ectoparasiticidal composition as claimed in claim 5 or 6, wherein the active ingredient is 1-perfluoropropyl-3-(3-trifluoromethylphenyl)-1,3-propanedione, or a sodium salt thereof.

10. An ectoparasiticidal composition as claimed in claim 5 or 6, wherein the active ingredient is 1-perfluoropropyl-3-(4-trifluoromethylphenyl)-1,3-propanedione, or a sodium salt thereof.

11. An ectoparasiticidal composition as claimed in claim 5 or 6, wherein the active ingredient is 1-perfluoropropyl-3-(2,4-difluorophenyl)-1,3-propanedione, or a sodium salt thereof.

12. An ectoparasiticidal composition as claimed in claim 5 or 6, wherein the actual ingredient is 1-perfluoropropyl-3-(3,5-bis(trifluoromethyl)phenyl)-1,3-propanedione, or a sodium salt thereof.

13. A process for preparing a compound of formula (I) as defined in claim 1, provided that when R is 5-bromo or 5-chlorothien-2-yl and $R^1$ is $C_3$ perfluoroalkyl, then the compound of formula (I) must be in the form of the sodium, lithium or potassium salt;
which comprises:

(A) acylating a compound of formula (II):

$$R\text{-}CO\text{-}CH_3 \qquad (II)$$

with an activated form of the acid of formula (III):

$$R^1CO_2H \qquad (III)$$

in the presence of a base sufficiently strong to generate the anion of the compound of formula (II); or

(B) hydrolyzing a compound of formula:

R-CO-CH-COR$^4$
        |
      COR$^1$

where $R^4$ represents $C_1$-$C_4$ perfluoroalkyl, and $R^1$ represents $C_3$-$C_6$ perfluorocycloalkyl, so as to provide a compound of formula (I) in which $R^1$ is $C_3$-$C_6$ perfluorocycloalkyl.

14. A process according to claim 13, wherein the R group has one or two substituents.